# EUROPÄISCHE PATENTSCHRIFT

(11) **EP 3 699 560 B1**
(45) Veröffentlichungstag und Bekanntmachung des Hinweises auf die Patenterteilung: **09.12.2026**
(21) Anmeldenummer: 19201941.2
(22) Anmeldetag: 08.10.2019
(51) Int. Cl.: G01G 19/50, G01G 21/28, G01G 23/37

(54) **VORRICHTUNG ZUR GEWICHTSMESSUNG**
DEVICE FOR WEIGHT MEASUREMENT
DISPOSITIF DE MESURE DE POIDS

(30) Priorität: 19.02.2019 DE 102019104165
(43) Veröffentlichungstag der Anmeldung: 26.08.2020
(73) Patentinhaber: seca gmbh & co. kg, 22089 Hamburg (DE)
(72) Erfinder: Vogel, Frederik, 20149 Hamburg (DE)
(74) Vertreter: Klickow & Wetzel PartGmbB

(56) Entgegenhaltungen:
- EP-A1- 2 700 917
- DE-B3- 102005 060 105
- DE-U1- 202013 010 582
- US-A1- 2005 155 246
- US-A1- 2009 166 096
- US-A1- 2018 035 918

## Beschreibung

Die Erfindung betrifft eine Vorrichtung zur Gewichtsmessung einer Person, wobei die Vorrichtung zur Gewichtsmessung mindestens eine digitale Anzeige- und Bedieneinheit aufweist.

Aus dem Stand der Technik bekannte Vorrichtungen zur Gewichtsmessung mit einer digitalen Anzeige- und Bedieneinheit sind beispielsweise als Säulenwaagen ausgebildet und weisen eine Basis auf, in deren Bereich eine Sensorik zur Gewichtserfassung angeordnet ist, sowie zumindest eine sich nach oben von der Basis erstreckende, beispielsweise als eine Säule ausgebildete Strebe. Am oberen Ende der Strebe ist eine digitale Anzeige- und Bedieneinheit angeordnet. Die digitale Anzeige- und Bedieneinheit ist dabei gemäß dem Stand der Technik werkseitig im Bereich des Kopfsegments entweder seitlich oder zentral in Bezug zur Waage positioniert und fest mit dem Kopfsegment verbunden.

Aus der DE 10 2005 060 105 B3 ist bereits eine Vorrichtung zur Gewichtsmessung bekannt, die mit einer Basis versehen ist, an deren Oberseite eine Wiegefläche angeordnet ist. Die entsprechende Vorrichtung weist darüber hinaus eine von der Basis ausgehende Strebe mit einem Kopfsegment auf. Im Bereich des Kopfsegmentes ist eine Anzeige-und Bedieneinheit angeordnet. Die Anzeige- und Bedieneinheit kann an unterschiedlichen Positionen angeordnet werden.

In der US 2009/0166096 A1 wird eine Personenwaage mit einer Anzeigeeinrichtung beschrieben. Eine separate Bedieneinrichtung kann an unterschiedlichen Positionen angeordnet werden. Insbesondere wird eine Anordnung auf einem Gestänge beschrieben.

Eine weitere Personenwaage ist aus der US 2018/0035918 A1 bekannt. Die entsprechende Waage besitzt eine Anzeigeeinrichtung. Eine separate Bedieneinrichtung ähnlich zu einem Smartphone kann an unterschiedlichen Positionen angeordnet werden. Insbesondere wird auch eine Anordnung auf einem teleskopierbaren Gestänge beschrieben.

Aus der EP 2 700 917 A1 ist eine Vorrichtung zur Messung der Zusammensetzung der Körpersubstanz eines Menschen bekannt. Es handelt sich hierbei um einen sogenannten BCA (Body Composition Analyzer). Im Bereich einer Basis sind Fußelektroden sowie eine Wiegeeinrichtung angeordnet. Eine Bedienungs- und Anzeigeeinheit ist in einem oberen Bereich einer Säule angeordnet. Links und rechts neben der Bedienungs- und Anzeigeeinheit sind Halterungen für Handelektroden positioniert. Die Einheit ist in Richtung auf einen Benutzer und von diesem weg in einer horizontalen Richtung positionierbar, um eine Anpassung an das Körpervolumen des Benutzers vorzunehmen.

In der US 2005/0155246 A1 wird eine Einrichtung sowohl zur Durchführung eines Wiegevorganges als auch einer Längenmessung beschrieben. Im Bereich einer säulenartigen Halterung erstreckt sich in einer Querrichtung eine Bedien- und Anzeigeeinheit.

Entsprechende Vorrichtungen zur Gewichtsmessung, wie beispielsweise Säulenwaagen, sind im Vergleich mit sonstigen medizintechnischen Geräten, die im Bereich von Arztpraxen verwendet werden, relativ groß.

Das medizinische Personal neigt deshalb dazu, die Vorrichtung zur Gewichtsmessung nahe den Wänden oder im Bereich von Ecken des Behandlungsraums aufzustellen. Hierdurch wird die Zugänglichkeit zur Anzeige- und Bedieneinheit für das medizinische Personal erschwert, da, zusätzlich zu einer im Bezug zur Positionierung der Vorrichtung zur Gewichtsmessung im Raum möglicherweise ungünstigen Anordnung der Anzeige- und Bedieneinheit an der Vorrichtung zur Gewichtsmessung, während der Messung der Patient auf der Waage steht und die Anzeige- und Bedieneinheit teilweise mit seinem Körper verdeckt.

Eine Aufgabe der Erfindung ist es daher, eine Vorrichtung der einleitend genannten Art derart anzugeben, dass unabhängig von der Positionierung der Vorrichtung zur Gewichtsmessung im Behandlungsraum für das medizinische Personal eine verbesserte Zugänglichkeit zu der Anzeige- und Bedieneinheit realisiert ist.

Diese Aufgabe wird erfindungsgemäß dadurch die Merkmalskombination von Patentanspruch 1 gelöst.

Eine erfindungsgemäße Vorrichtung zur Gewichtsmessung mit digitaler Anzeige- und Bedieneinheit umfasst einen Teil oder sämtliche der im Folgenden benannten Merkmale in allen ausführbaren Merkmalskombinationen.

Gemäß einer bevorzugten Ausführungsform einer erfindungsgemäßen Vorrichtung zur Gewichtsmessung weist diese eine Basis auf, in der die für die Erfassung des Körpergewichts einer Person erforderliche Sensorik angeordnet ist.

Auf der Oberseite der Basis ist eine Wiegefläche angeordnet, auf die sich eine Person zur Erfassung ihres Körpergewichts mit der Vorrichtung zur Gewichtsmessung stellt.

Im Bereich der Basis ist in einer Ausführungsform der Erfindung mindestens eine, beispielsweise als eine Säule ausgebildete Strebe angeordnet, die sich in Relation zu der auf der Oberseite der Basis angeordneten Wiegefläche nach oben hin erstreckt.

Die mindestens eine Strebe erstreckt sich vorzugsweise aus dem Bereich der Vorderseite der Basis oder aus einem der Vorderseite der Basis zugewandten Bereich der Seiten der Basis.

In einer bevorzugten Ausführungsform der Erfindung ist die Vorrichtung zur Gewichtsmessung als eine Säulenwaage ausgebildet.

In einer Ausführungsform einer Vorrichtung zur Gewichtsmessung ausgebildet als eine Säulenwaage erstreckt sich eine als eine Säule ausgebildete Strebe mittig an der Vorderseite der Basis in Relation zur der auf der Oberseite der Basis angeordneten Wiegefläche senkrecht nach oben.

An der Oberseite der mindestens einen Strebe ist in einer bevorzugten Ausführungsform einer erfindungsgemäßen Vorrichtung zur Gewichtsmessung ein Kopfsegment angeordnet, das eine wesentliche horizontale Ausdehnungskomponente aufweist.

In einer bevorzugten Ausführungsform der Erfindung erstreckt sich das Kopfsegment quer zu Längsrichtung der Strebe.

In einer besonders bevorzugten Ausführungsform der Erfindung ist die Strebe mittig an der Vorderseite der Basis angeordnet und das Kopfsegment ist derart an der Oberseite der Strebe angeordnet, dass dieses sich oberhalb der Strebe seitlich nach links und rechts erstreckt. Die in Form einer Säule ausgebildete Strebe und das Kopfsegment sind in dieser Ausführungsform miteinander verbunden hammerartig ausgebildet.

In einer weiteren Ausführungsform einer erfindungsgemäßen Vorrichtung zur Gewichtsmessung weist diese eine oder mehrere Streben auf, die sich aus dem Bereich der Basis nach oben hin erstecken und die durch eine geschwungene Form oder durch die Verbindung mit weiteren Strebeelementen ein Profil bilden, das im Bereich des Kopfsegmentes eine horizontale Ausdehnungskomponente aufweist.

In einer bevorzugten Ausführungsform der vorstehend benannten Vorrichtung zur Gewichtsmessung ist das mithilfe der mindestens einen Strebe realisierte Profil etwa U-förmig bzw. bogenförmig und entweder mit fließenden Übergängen oder eckig ausgeführt, wobei das U-Profil sich in Richtung der Basis öffnet.

In einer Ausführungsform der Erfindung ist mithilfe der mindestens einen Strebe ein Handlauf realisiert.

In einer Ausführungsform der Erfindung ist der Handlauf zumindest im Bereich des Kopfsegments als ein Kreissegment ausgebildet.

Eine erfindungsgemäße Vorrichtung zur Gewichtsmessung weist zumindest eine digitale Anzeige- und Bedieneinheit auf, die im Bereich des Kopfsegments angeordnet ist. Erfindungsgemäß ist im Bereich des Kopfsegments zumindest eine Aufnahme- und Verbindungseinrichtung angeordnet, mit der mindestens eine digitale Anzeige- und Bedieneinheit lösbar derart verbindbar ist, dass eine Positionierung der Anzeige- und Bedieneinheit in mindestens zwei verschiedenen Positionen im Bereich des Kopfsegments ermöglicht ist.

In einer erfindungsgemäßen Ausführungsform einer Vorrichtung zur Gewichtsmessung sind dazu zwei Aufnahme- und Verbindungseinrichtungen an mindestens zwei festen Positionen im Bereich des Kopfsegments der Vorrichtung zur Gewichtsmessung angeordnet, mit denen eine digitale Anzeige- und Bedieneinheit verbindbar ist.

In einer bevorzugten Ausführungsform einer Vorrichtung zur Gewichtsmessung weist diese im Kopfsegment zwei Aufnahme- und Verbindungseinrichtungen auf, von denen eine Aufnahme- und Verbindungeinrichtung in einem linken Bereich des Kopfsegments und eine zweite Aufnahme- und Verbindungseinheit in einem rechten Bereich des Kopfsegments angeordnet ist.

In einer weiteren erfindungsgemäßen Ausführungsform einer Vorrichtung zur Gewichtsmessung weist diese im Kopfsegment zumindest eine Aufnahme- und Verbindungseinrichtung auf, die sich in Längsrichtung des Kopfsegments erstreckt und mit der eine digitale Anzeige- und Bedieneinheit in verschiedenen Positionen verbindbar ist.

In einer besonders bevorzugten Ausführungsform einer erfindungsgemäßen Vorrichtung zur Gewichtsmessung mit zumindest einer sich in Längsrichtung des Kopfsegment erstreckenden Aufnahme- und Verbindungeinrichtung, ist die digitale Anzeige-und Bedieneinheit innerhalb des Erstreckungsbereiches der Aufnahme- und Verbindungeinrichtung frei mit dieser verbindbar, in dem Sinne, dass außer den durch die äußeren Abmessungen der Aufnahme- und Verbindungseinrichtungen vorgegebenen Endpositionen in Längsrichtung der Aufnahme- und Verbindungeinrichtung keine Positionsvorgaben für die Anzeige- und Bedieneinheit gegeben sind.

In einer erfindungsgemäßen Ausführungsform einer Vorrichtung zur Gewichtsmessung mit mindestens einer horizontal erstreckten Aufnahme- und Verbindungseinrichtung ist diese als eine Schiene ausgebildet, mit der die Anzeige- und Bedieneinheit verbindbar ist und auf der die digitale Anzeige- und Bedieneinheit frei verschiebbar ist.

In einer weiteren Ausführungsform einer erfindungsgemäßen Vorrichtung zur Gewichtsmessung weist die als eine horizontal verlaufende Schiene ausgebildete Aufnahme- und Verbindungseinrichtung Rastpunkte auf, die in horizontaler Richtung feste Positionen für die mindestens eine Anzeige- und Bedieneinheit vorgeben.

In einer weiteren erfindungsgemäßen Ausführungsform einer Vorrichtung zur Gewichtsmessung ist die mindestens eine Aufnahme- und Verbindungeinrichtung als ein Klettband ausgebildet, dass sich horizontal im Bereich des Kopfsegments erstreckt und mit dem eine digitale Anzeige- und Bedieneinheit entsprechend mit einem korrespondierend zu dem Klettband ausgebildeten Klettelement verbindbar ist.

In einer weiteren Ausführungsform einer erfindungsgemäßen Vorrichtung zur Gewichtsmessung ist die mindestens eine Aufnahme- und Verbindungseinrichtung als ein sich horizontal im Bereich des Kopfsegments erstreckendes Magnetband oder als eine ferromagnetische Schiene oder als in horizontaler Richtung aneinandergereihte magnetische Elemente oder als in horizontaler Richtung aneinandergereihte ferromagnetische Elemente ausgebildet, sodass eine digitale Anzeige- und Bedieneinheit mit einem korrespondierenden magnetischen oder ferromagnetischen Verbindungselement mit der Aufnahme- und Verbindungseinrichtung verbindbar ist.

In einer Ausführungsform einer erfindungsgemäßen Vorrichtung zur Gewichtsmessung sind nicht mit einer Anzeige- und Bedieneinheit verbundene Aufnahme- und Verbindungseinrichtungen oder nicht mit einer Anzeige- und Bedieneinheit verbundene Bereiche einer Aufnahme- und Verbindungseinrichtungen mit einer oder mehreren Abdeckungen verschließbar.

Eine Fixierung der Anzeige- und Bedieneinheit an der mindestens einen Aufnahme- und Verbindungseinrichtung im Bereich des Kopfsegments kann in verschiedenen Ausführungsformen einer erfindungsgemäßen Vorrichtung zur Gewichtsmessung vorzugsweise durch Verschrauben, magnetische Kräfte, Klettelemente oder durch ein Einrasten korrespondierender Rastvorrichtungen an der Anzeige- und Bedieneinheit und der mindestens einen Aufnahme- und Verbindungseinrichtung erfolgen.

Eine digitale Anzeige- und Bedieneinheit einer erfindungsgemäßen Vorrichtung zur Gewichtsmessung weist zumindest ein Display und zumindest eine Eingabevorrichtung zur Erfassung von Benutzereingaben auf.

In einer erfindungsgemäßen Ausführungsform einer Vorrichtung zur Gewichtsmessung dient die digitale Anzeige- und Bedieneinheit der Bedienung der Vorrichtung zur Gewichtsmessung beispielsweise dem Einschalten des Messgeräts, dem Starten einer Messung, dem Tarieren des Systems, dem Umschalten zwischen Gewichtseinheiten und/oder der Ansteuerung zusätzlicher Messfunktionen. Darüber hinaus dient die digitale Anzeige- und Bedieneinheit dem Anzeigen von Messwerten und/oder den vom Bediener gewählten Einstellungen.

In einer erfindungsgemäßen Ausführungsform einer Vorrichtung zur Gewichtsmessung ist die digitale Anzeige- und Bedieneinheit kabelgebunden mit der Vorrichtung zur Gewichtsmessung verbunden.

In einer weiteren Ausführungsform der Erfindung ist die digitale Anzeige- und Bedieneinheit kabellos mit der Vorrichtung zur Gewichtsmessung verbunden. Eine Anbindung der digitalen Anzeige- und Bedieneinheit an die korrespondierende Elektronik im übrigen Teil der Vorrichtung zur Gewichtsmessung ist dabei bevorzugt als eine drahtlose Übertragungsschnittstelle, beispielsweise ausgebildet als eine WiFi- oder eine Bluetooth-Schnittstelle, ausgeführt.

**In** einer Ausführungsform der Erfindung weist die mindestens eine digitale Anzeige-und Bedieneinheit einen integrierten Energiespeicher auf, der vorzugsweise als ein Akkumulator ausgebildet ist.

Die drahtlose Schnittstelle der digitalen Anzeige- und Bedieneinheit ist in einer Ausführungsform einer erfindungsgemäßen Vorrichtung zur Gewichtsmessung darüber hinaus verwendbar, um die Vorrichtung zur Gewichtsmessung in ein digitales Patientensystem zu integrieren.

In einer bevorzugten Ausführungsform weist die Vorrichtung zur Gewichtsmessung mindestens eine zusätzliche Funktionskomponente auf. Die zusätzliche Funktionskomponente ist beispielsweise als eine Längenmesseinrichtung realisiert, die vorzugsweise als ein Längenmessstab ausgebildet ist.

In einer Ausführungsform der Erfindung weist diese eine Längenmesseinrichtung auf, die vertikal positionierbar ist.

In einer Ausführungsform einer erfindungsgemäßen Vorrichtung zur Gewichtsmessung ist der Längenmessstab im Bereich einer Strebe der Vorrichtung zur Gewichtsmessung angeordnet. Besonders bevorzugt ist die Vorrichtung zur Gewichtsmessung dabei als eine Säulenwaage ausgebildet und die Längenmesseinrichtung ist im Bereich der Säule angeordnet.

In einer erfindungsgemäßen Ausführungsform einer Vorrichtung zur Gewichtsmessung weist der Längenmessstab einen verschwenkbaren Kopfanschlag auf.

In einer erfindungsgemäßen Ausführungsform einer Vorrichtung zur Gewichtsmessung ist mithilfe des Längenmessstabs ein digitales Längenmesssystem implementiert, mit dem die Körpergröße einer Person auf der Waage bestimmbar ist.

In einer erfindungsgemäßen Ausführungsform einer Vorrichtung zur Gewichtsmessung ist der mithilfe des digitalen Längenmesssystems erfasste Messwert der Körpergröße der Person auf dem Display der Anzeige- und Bedieneinheit darstellbar.

In einer erfindungsgemäßen Ausführungsform einer Vorrichtung zur Gewichtsmessung ist zusätzlich eine unmittelbare Ablesung des Längenmesswertes an einer Skala im Bereich des Längenmessstabes möglich.

In einer erfindungsgemäßen Ausführungsform einer Vorrichtung zur Gewichtsmessung weist diese zwei digitale Anzeige- und Bedieneinheiten auf, die im Bereich des Kopfsegmentes an verschiedenen Positionen mit diesem verbunden sind.

Vorzugsweise sind diese zwei digitalen Anzeige- und Bedieneinheiten dabei derart angeordnet, dass eine erste digitale Anzeige- und Bedieneinheit auf der von der linken Seite der Vorrichtung zur Gewichtsmessung zugänglichen Seite angeordnet ist und eine zweite digitale Anzeige- und Bedieneinheit derart angeordnet ist, dass diese von der rechten Seite der Vorrichtung zur Gewichtsmessung zugänglich ist.

In einer Ausführungsform einer erfindungsgemäßen Vorrichtung zur Gewichtsmessung weist diese einen Scanner auf, mit dem beispielsweise Barcodes und/oder QR-Codes zur Identifikation eines Patienten bzw. zur Erfassung einer Patienten-ID erfassbar sind.

In den im Folgenden beschriebenen Figuren sind beispielhafte Ausführungsformen der erfindungsgemäßen Vorrichtung zur Gewichtsmessung dargestellt. Es zeigen
- Figur 1:: eine perspektivische Ansicht einer erfindungsgemäßen Vorrichtung zur Gewichtsmessung ausgebildet als eine Säulenwaage mit einer digitalen Anzeige- und Bedieneinheit verbunden mit einem hammerartig ausgebildeten Modul aus Kopfsegment und Säule,
- Figur 2:: eine perspektivische Ansicht eines Ausschnitts im Kopfbereich der erfindungsgemäßen Säulenwaage aus Figur 1,
- Figur 3:: eine perspektivische Ansicht der in den Figuren 1 und 2 dargestellten Säulenwaage in einem Ausschnitt des Kopfbereiches, wobei die digitale Anzeige- und Bedieneinheit nun auf der linken Seite des Kopfsegments angeordnet ist,
- Figur 4:: perspektivische Ansichten des Ausschnitts im Kopfbereich der in Figur 1 dargestellten Säulenwaage zur Veranschaulichung der benötigten Schritte zum Austausch der Positionen der digitalen Anzeige- und Bedieneinheit,
- Figur 5:: eine perspektivische Ansicht einer erfindungsgemäß ausgebildeten digitalen Anzeige- und Bedieneinheit,
- Figur 6:: eine perspektivische Ansicht einer weiteren erfindungsgemäß ausgebildeten digitalen Anzeige- und Bedieneinheit und
- Figur 7:: drei perspektivische Ansichten einer alternativen Ausführungsform einer erfindungsgemäßen Vorrichtung zur Gewichtsmessung.

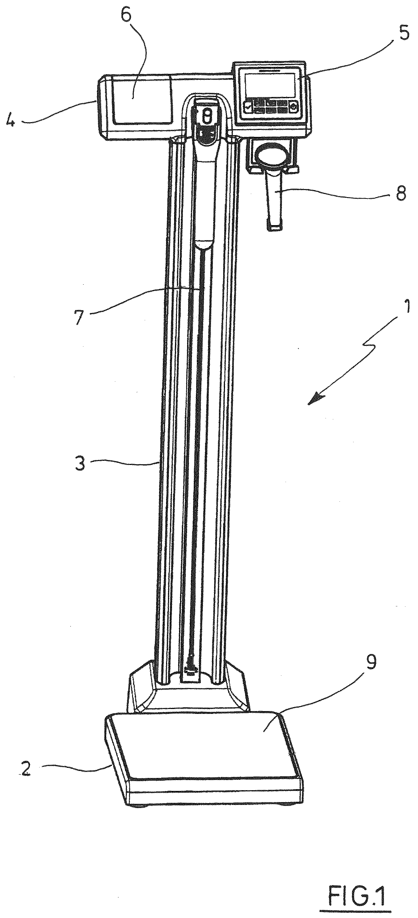

Figur 1 zeigt eine perspektivische Ansicht einer erfindungsgemäßen Vorrichtung zur Gewichtsmessung (1) ausgebildet als eine Säulenwaage. Die Vorrichtung zur Gewichtsmessung (1) weist eine Basis (2), eine sich nach oben hin von der Basis erstrekkende Säule (3) und ein am oberen Ende der Säule (3) angeordnetes Kopfsegment (4) auf. Das Kopfsegment (4) erstreckt sich quer zur Längsrichtung der Säule (3), sodass die von der Säule (3) und dem Kopfsegment (4) gebildete Einheit in einer hammerartigen Form ausgebildet ist.

Im Bereich des Kopfsegments (4) sind zwei Aufnahme- und Verbindungseinrichtungen (6) angeordnet, von denen eine erste Aufnahme- und Verbindungseinrichtung (6) auf der linken Seite des Kopfsegments (4) und eine zweite Aufnahme- und Verbindungseinrichtung (6) auf der rechten Seite des Kopfsegments (4) angeordnet ist.

Die auf der linken Seite des Kopfsegments (4) angeordnete Aufnahme- und Verbindungseinrichtung (6) ist mithilfe einer Abdeckung verschlossen. Die auf der rechten Seite des Kopfsegments (4) angeordnete Aufnahme- und Verbindungseinrichtung (6) ist mit einer Anzeige- und Bedieneinheit (5) verbunden. Im Bereich der Säule (3) weist die dargestellte Ausführungsform einer erfindungsgemäßen Vorrichtung zur Gewichtsmessung (1) eine Längenmesseinrichtung (7) auf.

Unterhalb der Anzeige- und Bedieneinheit (5) ist ein Scanner (8) mithilfe einer korrespondierenden Halterungsvorrichtung gehaltert.

Auf der Oberseite der Basis (2) der erfindungsgemäßen Ausführungsform einer Vorrichtung zur Gewichtsmessung (1) ist eine Wiegefläche (9) angeordnet.

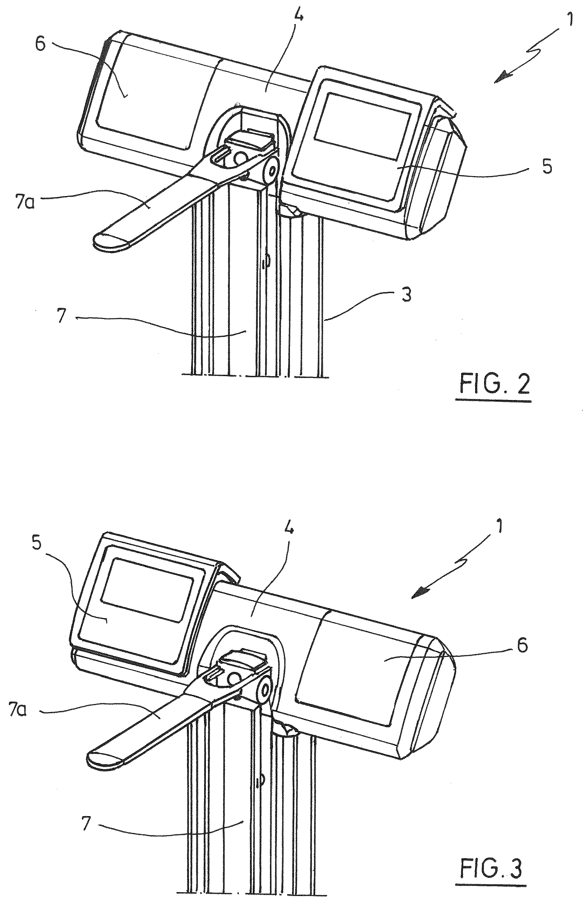

In Figur 2 ist ausschnittsweise eine perspektivische Darstellung der in Figur 1 dargestellten Vorrichtung zur Gewichtsmessung (1) im Bereich des Kopfsegments (4) dargestellt. Weiterhin weist die Längenmesseinrichtung (7) der dargestellten erfindungsgemäßen Ausführungsform einer Vorrichtung zur Gewichtsmessung (1) einen verschwenkbaren Kopfanschlag (7a) auf.

Figur 3 zeigt einen Ausschnitt einer perspektivischen Ansicht der in den Figuren 1 und 2 dargestellten Vorrichtung zur Gewichtsmessung (1), wobei die Anzeige- und Bedieneinheit (5) nun mit der Aufnahme- und Verbindungseinrichtung (6) auf der linken Seite des Kopfsegments (4) verbunden ist und die Aufnahme- und Verbindungseinrichtung (6) auf der rechten Seite des Kopfsegments (4) mit einer Abdeckung verschlossen ist.

Während die Anzeige- und Bedieneinheit (5) der in den Figuren 1 und 2 dargestellten Konfiguration einer erfindungsgemäßen Vorrichtung zur Gewichtsmessung (1) von der rechten Seite der Vorrichtung zur Gewichtsmessung (1) eine erhöhte Zugänglichkeit aufweist, ist diese bei der in Figur 3 dargestellten Konfiguration einer erfindungsgemäßen Vorrichtung zur Gewichtsmessung (1) von der linken Seite der Vorrichtung zur Gewichtsmessung (1) gegeben.

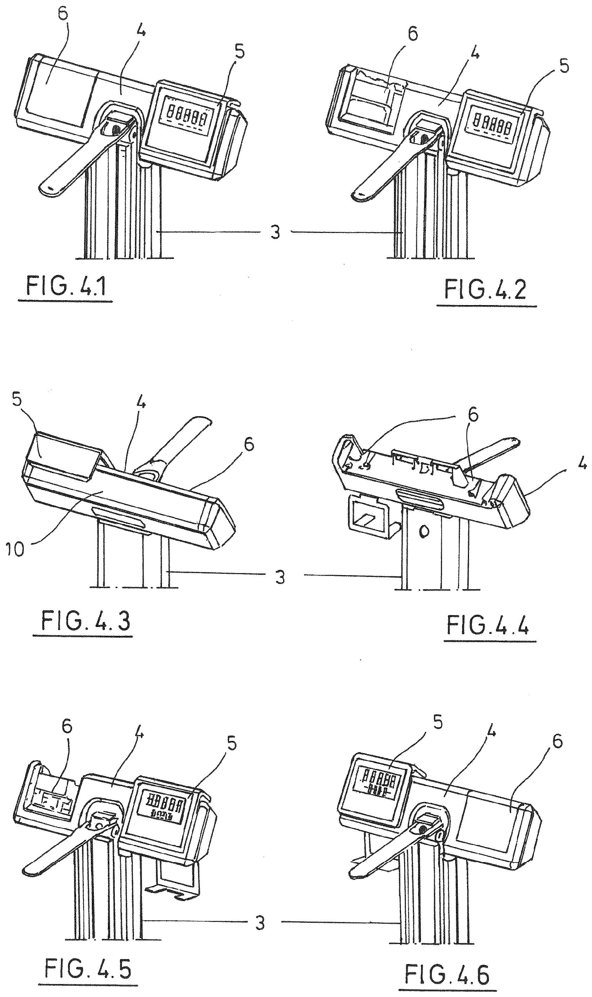

Figur 4 zeigt in sechs Darstellungen verschiedene Schritte, die für den Wechsel der Position der digitalen Anzeige- und Bedieneinheit (5) der in den vorigen Figuren dargestellten erfindungsgemäßen Ausführungsform einer Vorrichtung zur Gewichtsmessung (1) erforderlich sind.

Figur 4.1 zeigt die auch in Figur 2 dargestellte Konfiguration einer erfindungsgemäßen Ausführungsform einer Vorrichtung zur Gewichtsmessung (1), in der die linke Aufnahme- und Verbindungseinrichtung (6) mit einer Abdeckung verschlossen ist und die rechte Aufnahme- und Verbindungseinrichtung (6) mit einer digitalen Anzeige- und Bedieneinheit (5) verbunden ist.

Bei dem in Figur 4.2 dargestellten Schritt ist die Abdeckung von der linken Aufnahme- und Verbindungseinrichtung (6) entfernt.

Figur 4.3 zeigt die erfindungsgemäße Ausführungsform einer Vorrichtung zur Gewichtsmessung (1) in einer perspektivischen Ansicht von der Rückseite. Auf der Rückseite des Gehäuses des Kopfsegments (4) ist ein Gehäuseteil (10) lösbar montiert.

Figur 4.4 zeigt die in Figur 4.3 dargestellte Säulenwaage von der Rückseite, wobei die Abdeckung der Aufnahme- und Verbindungseinrichtung (6), die digitale Anzeige- und Bedieneinheit (5) und das Gehäuseteil (10) vom Kopfsegment (4) demontiert sind. Durch das demontierbare rückwärtig angeordnete Gehäuseteil (10) ist ein Positionswechsel der digitalen Anzeige- und Bedieneinheit (5) auch im Falle einer kabelgebundenen Verbindung von digitaler Aufnahme- und Verbindungseinrichtung (5) mit der korrespondierenden Elektronik der übrigen Vorrichtung zur Gewichtsmessung (1) ohne ein Lösen der kabelgebundenen Verbindung möglich. Zudem ist ein die digitale Anzeige- und Bedieneinheit (5) mit der Vorrichtung zur Gewichtsmessung (1) verbindendes Kabel in beiden vollständig montierten Positionen der Anzeige- und Bedieneinheit (5) im Bereich des Kopfsegments (4) im Gehäuse verborgen.

Figur 4.6 zeigt die erfindungsgemäße Ausführungsform einer Vorrichtung zur Gewichtsmessung (1) nach dem abgeschlossenen Positionswechsel der Anzeige- und Bedieneinheit (5) von der rechten Aufnahme- und Verbindungseinrichtung (6) in die linke Aufnahme- und Verbindungseinrichtung (6). Die rechte Aufnahme- und Verbindungseinrichtung (6) ist nun mit der Abdeckung verschlossen.

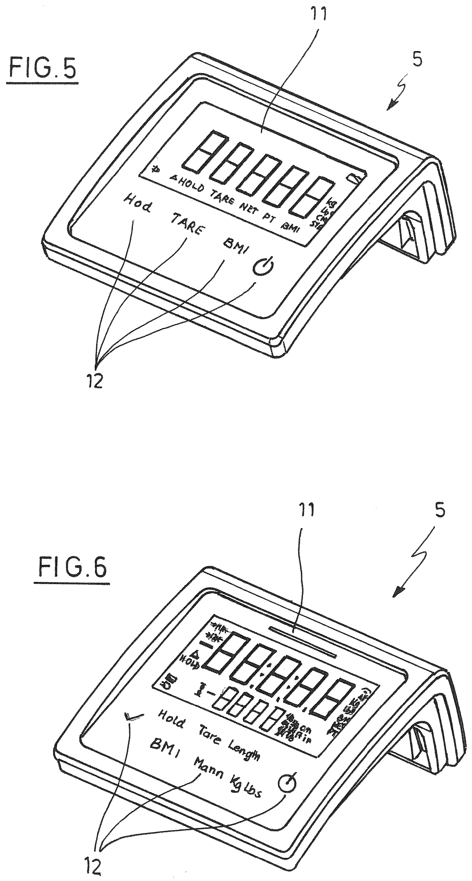

**In** Figur 5 ist eine erfindungsgemäße Ausführungsform einer digitalen Anzeige- und Bedieneinheit (5) einer Vorrichtung zur Gewichtsmessung (1) in einer perspektivischen Ansicht dargestellt. Die digitale Anzeige- und Bedieneinheit (5) weist ein Display (11) sowie vier unterhalb des Displays (11) angeordnete Eingabevorrichtungen (12) auf. Die Eingabevorrichtungen (12) sind als berührungsempfindliche Touch-Elemente ausgebildet. Das Display (11) weist eine Segmentanzeige zur Darstellung von fünf Ziffern zur Anzeige erfasster Messwerte auf. Darüber hinaus sind dem Display (11) der digitalen Anzeige- und Bedieneinheit (5) Informationen über vom Bediener gewählte Optionen sowie der Ladezustand eines in die digitale Anzeige- und Bedieneinheit (5) integrierten Akkus entnehmbar.

In Figur 6 ist eine Ausführungsform einer digitalen Anzeige- und Bedieneinheit (5) einer erfindungsgemäßen Ausführungsform einer Vorrichtung zur Gewichtsmessung (1) mit einer erweiterten Funktionalität dargestellt. Zusätzlich zum Gewichtsmesswert ist ein erfasster Längenmesswert darstellbar. Weiterhin ist über zusätzliche Eingabevorrichtungen (12) ein Menü aufrufbar, eine Bioimpedanzmessung startbar, eine Längenmessung durchführbar und eine Auswahl bestätigbar. Darüber hinaus weist die dargestellte Anzeige- und Bedieneinheit (5) eine WiFi-Schnittstelle auf, deren Verbindungsstatus auf dem Display (11) der Anzeige- und Bedieneinheit (5) ablesbar ist.

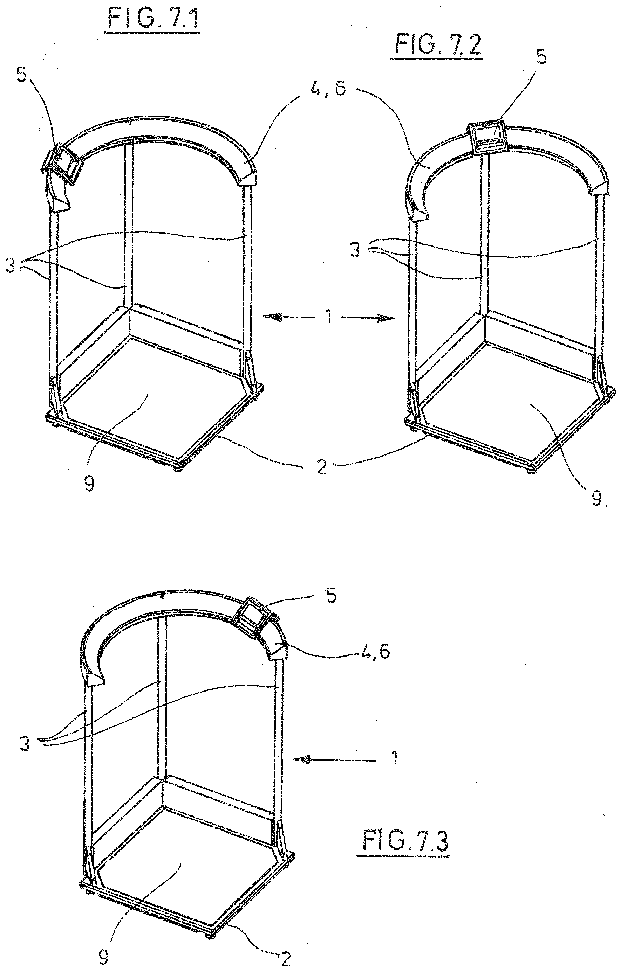

Figur 7 zeigt in den Figuren 7.1 bis 7.3 drei perspektivische Darstellungen einer weiteren erfindungsgemäßen Ausführungsform einer Vorrichtung zur Gewichtsmessung (1). Das Kopfsegment (4) ist als eine halbkreisförmig umlaufende Schiene ausgebildet, auf der eine Anzeige- und Bedieneinheit (5) stufenlos verschiebbar ist. Durch die Stufenlose Verschiebbarkeit weist das Kopfsegment (4) entlang seiner Erstreckung eine Vielzahl von Aufnahme- und Verbindungseinrichtungen (6) auf bzw. ist in seiner Gesamtheit eine einzige Aufnahme- und Verbindungseinrichtung (6) mit einer faktisch unendlichen Anzahl an Positionen, an denen die Anzeige- und Bedieneinheit (5) mit dieser funktional verbindar ist. Die Vorrichtung zur Gewichtsmessung (1) weist darüber hinaus drei Streben (3) auf, die das Kopfsegment (4) tragen.

Gemäß der Darstellung in Figur 7.1 ist die Anzeige- und Bedieneinheit (5) in einem Bereich auf der linken Seite des Kopfsegments (4) angeordnet, in Figur 7.2 ist die Anzeige- und Bedieneinheit (5) in einem Bereich in der Mitte des Kopfsegments (4) angeordnet und in Figur 7.3 ist die Anzeige- und Bedieneinheit (5) in einem Bereich auf der rechten Seite des Kopfsegments (4) angeordnet.

Alternativ zu der vorstehend beschriebenen Ausführungsform einer erfindungsgemäßen Vorrichtung zur Gewichtsmessung als Säulenwaage sind ausdrücklich auch Ausführungsformen mit abweichenden Varianten der Anordnung von Streben und Kopfsegment entsprechend der Beschreibung umfasst.

## Patentansprüche

1. Vorrichtung zur Gewichtsmessung (1), aufweisend eine Basis (2), an deren Oberseite eine Wiegefläche (9) angeordnet ist, weiterhin aufweisend mindestens eine sich nach oben von der Basis (2) erstreckende Strebe (3) mit einem Kopfsegment (4), das eine wesentliche horizontale Ausdehnungskomponente aufweist, weiterhin aufweisend mindestens eine digitale Anzeige- und Bedieneinheit (5), die im Bereich des Kopfsegments (4) angeordnet ist, wobei die mindestens eine digitale Anzeige- und Bedieneinheit (5) mit mindestens einer im Bereich des Kopfsegments (4) angeordneten Aufnahme- und Verbindungseinrichtung (6) derart verbindbar ist, dass eine Positionierung der Anzeige- und Bedieneinheit (5) in mindestens zwei verschiedenen Positionen im Bereich des Kopfsegments (4) ermöglicht ist und bei der sich die Aufnahme- und Verbindungseinrichtung (6) derart in horizontaler Richtung erstreckt, dass die digitale Anzeige- und Bedieneinheit (5) an verschiedenen Positionen mit der Aufnahme- und Verbindungseinrichtung (6) verbindbar ist und wobei sich das Kopfsegment (4) quer zu einer Längsachse der Strebe (3) erstreckt, **dadurch gekennzeichnet, dass** die mindestens eine Anzeige- und Bedieneinheit (5) im Bereich des Kopfsegments (4) in je zumindest einer Position im linken und im rechten Bereich des Kopfsegments (4) mit mindestens einer Aufnahme- und Verbindungseinrichtung (6) verbindbar ist.

2. Vorrichtung zur Gewichtsmessung (1) nach mindestens einem der vorstehenden Ansprüche, **dadurch gekennzeichnet, dass** die mindestens eine digitale Anzeige- und Bedieneinheit (5) in mindestens zwei fest vorgegebenen Positionen im Bereich des Kopfsegments (4) mit der Vorrichtung zur Gewichtsmessung (1) verbindbar ist.

3. Vorrichtung zur Gewichtsmessung (1) nach zumindest einem der vorstehenden Ansprüche, **dadurch gekennzeichnet, dass** die mindestens eine digitale Anzeige- und Bedieneinheit (5) kabelgebunden mit der korrespondierenden Elektronik der Vorrichtung zur Gewichtsmessung (1) verbindbar ist.

4. Vorrichtung zur Gewichtsmessung (1) nach mindestens einem der Ansprüche 1 bis 3, **dadurch gekennzeichnet, dass** die mindestens eine digitale Anzeige-und Bedieneinheit (5) kabellos mit der korrespondierenden Elektronik der Vorrichtung zur Gewichtsmessung (1) verbindbar ist.

5. Vorrichtung zur Gewichtsmessung (1) nach mindestens einem der vorstehenden Ansprüche, **dadurch gekennzeichnet, dass** die digitale Anzeige- und Bedieneinheit (5) im Bereich des Kopfsegments (4) innerhalb von horizontalen Abmessungen der mindestens einen Aufnahme- und Verbindungseinrichtung (6) frei positionierbar ist.

6. Vorrichtung zur Gewichtsmessung (1) nach mindestens einem der vorstehenden Ansprüche, **dadurch gekennzeichnet, dass** die Aufnahme- und Verbindungseinrichtung (6) als eine horizontal verlaufende Schiene ausgebildet ist, mit der die digitale Anzeige- und Bedieneinheit (5) verbindbar ist.

7. Vorrichtung zur Gewichtsmessung (1) nach mindestens einem der vorstehenden Ansprüche, **dadurch gekennzeichnet, dass** die mindestens eine Aufnahme- und Verbindungseinrichtung (6) als ein sich in horizontaler Richtung erstreckendes Klettband realisiert ist.

8. Vorrichtung zur Gewichtsmessung (1) nach mindestens einem der vorstehenden Ansprüche, **dadurch gekennzeichnet, dass** die mindestens eine Aufnahme- und Verbindungseinrichtung (6) als ein sich in horizontaler Richtung erstreckendes magnetisches oder ferromagnetisches Element ausgebildet ist.

9. Vorrichtung zur Gewichtsmessung (1) nach mindestens einem der vorstehenden Ansprüche, **dadurch gekennzeichnet, dass** diese eine Längenmesseinrichtung (7) aufweist.

10. Vorrichtung zur Gewichtsmessung (1) nach Anspruch 9, **dadurch gekennzeichnet, dass** die Längenmesseinrichtung (7) als ein Längenmessstab ausgebildet ist.

11. Vorrichtung zur Gewichtsmessung (1) nach mindestens einem der Ansprüche 9 und 10 , **dadurch gekennzeichnet, dass** die Längenmesseinrichtung (7) vertikal positionierbar ist.

12. Vorrichtung zur Gewichtsmessung (1) nach mindestens einem der Ansprüche 9 bis 11, **dadurch gekennzeichnet, dass** die Längenmesseinrichtung (7) einen Kopfanschlag (7a) aufweist.

13. Vorrichtung zur Gewichtsmessung (1) nach mindestens einem der vorstehenden Ansprüche, **dadurch gekennzeichnet, dass** diese als eine Säulenwaage ausgebildet ist.

## Claims

1. Device for weight measurement (1), comprising a base (2) on the upper surface of which a weighting surface (9) is arranged, further comprising at least one strut (3) extending upward from the base (2) and having a head segment (4) that exhibits a substantial horizontal component of extension, and further comprising at least one digital display and control unit (5), which is arranged in the area of the head segment (4), wherein the at least one digital display and control unit (5) can be connected to at least one mounting and connecting device (6) arranged in the area of the head segment (4) in such a way that the display and control unit (5) can be positioned in at least two different positions in the area of the head segment (4), and wherein the mounting and connecting device (6) extends in the horizontal direction such that the digital display and control unit (5) can be connected to the mounting and connecting device (6) at different positions, and wherein the head segment (4) extends transversely to a longitudinal axis of the strut (3), **characterized in that** the at least one display and control unit (5) can be connected to at least one mounting and connecting device (6) in the area of the head segment (4), in at least one position each in the left and right regions of the head segment (4).

2. Device for weight measurement (1) according to at least one of the preceding claims, **characterized in that** the at least one digital display and control unit (5) can be connected to the device for weight measurement (1) in at least two fixed positions in the area of the head segment (4).

3. Device for weight measurement (1) according to at least one of the preceding claims, **characterized in that** the at least one digital display and control unit (5) can be connected via a cable to the corresponding electronics of the device for weight measurement (1).

4. Device for weight measurement (1) according to at least one of claims 1 to 3, **characterized in that** the at least one digital display and control unit (5) can be connected wirelessly to the corresponding electronics of the device for weight measurement (1).

5. Device for weight measurement (1) according to at least one of the preceding claims, **characterized in that** the digital display and control unit (5) can be freely positioned in the area of the head segment (4) within the horizontal dimensions of the at least one mounting and connecting device (6).

6. Device for weight measurement (1) according to at least one of the preceding claims, **characterized in that** the mounting and connecting device (6) is designed as a horizontally extending rail to which the digital display and control unit (5) can be connected.

7. Device for weight measurement (1) according to at least one of the preceding claims, **characterized in that** the at least one mounting and connecting device (6) is implemented as a hook-and-loop fastener extending in the horizontal direction.

8. Device for weight measurement (1) according to at least one of the preceding claims, **characterized in that** the at least one mounting and connecting device (6) is configured as a magnetic or ferromagnetic element extending in a horizontal direction.

9. Device for weight measurement (1) according to at least one of the preceding claims, **characterized in that** it comprises a length-measuring device (7).

10. Device for weight measurement (1) according to claim 9, **characterized in that** the length-measuring device (7) is configured as a length-measuring rod.

11. Device for weight measurement (1) according to at least one of claims 9 and 10, **characterized in that** the length-measuring device (7) is vertically positionable.

12. Device for weight measurement (1) according to at least one of claims 9 to 11, **characterized in that** the length-measuring device (7) comprises a head stop (7a).

13. Device for weight measurement (1) according to at least one of the preceding claims, **characterized in that** it is configured as a column scale.

## Revendications

1. Dispositif de mesure de poids (1) comportant une base (2), sur le côté supérieur de laquelle est disposée une surface de pesée (9), comportant par ailleurs au moins une entretoise (3) s'étendant vers le haut depuis la base (2) avec un segment de tête (4) qui présente une composante d'expansion sensiblement horizontale, comportant par ailleurs au moins une unité d'affichage et de commande numérique (5) qui est disposée dans la zone du segment de tête (4), l'au moins une unité d'affichage et de commande numérique (5) pouvant être reliée à au moins un système de réception et de liaison (6) disposé dans la zone du segment de tête (4) de telle manière qu'un positionnement de l'unité d'affichage et de commande (5) dans au moins deux positions différentes dans la zone du segment de tête (4) est rendu possible et dans lequel le système de réception et de liaison (6) s'étend de telle manière dans une direction horizontale que l'unité d'affichage et de commande numérique (5) peut être reliée au système de réception et de liaison (6) sur différentes positions, et le segment de tête (4) s'étendant transversalement à un axe longitudinal de l'entretoise (3), **caractérisé en ce que** l'au moins une unité d'affichage et de commande (5) peut être reliée à au moins un système de réception et de liaison (6) dans la zone du segment de tête (4), dans respectivement au moins une position dans la zone gauche et dans la zone droite du segment de tête (4).

2. Dispositif de mesure de poids (1) selon la revendication précédente, **caractérisé en ce que** l'au moins une unité d'affichage et de commande numérique (5) peut être reliée au dispositif de mesure de poids (1) dans au moins deux positions prédéfinies de manière fixe dans la zone du segment de tête (4).

3. Dispositif de mesure de poids (1) selon au moins l'une des revendications précédentes, **caractérisé en ce que** l'au moins une unité d'affichage et de commande numérique (5) peut être reliée de manière filaire à l'électronique correspondante du dispositif de mesure de poids (1).

4. Dispositif de mesure de poids (1) selon au moins l'une des revendications 1 à 3, **caractérisé en ce que** l'au moins une unité d'affichage et de commande numérique (5) peut être reliée sans fil à l'électronique correspondante du dispositif de mesure de poids (1).

5. Dispositif de mesure du poids (1) selon au moins l'une des revendications précédentes, **caractérisé en ce que** l'unité d'affichage et de commande numérique (5) peut être positionnée librement dans la zone du segment de tête (4) à l'intérieur de dimensions horizontales de l'au moins un système de réception et de liaison (6).

6. Dispositif de mesure de poids (1) selon au moins l'une des revendications précédentes, **caractérisé en ce que** le système de réception et de liaison (6) est formé comme un rail s'étendant horizontalement, auquel l'unité d'affichage et de commande numérique (5) peut être reliée.

7. Dispositif de mesure de poids (1) selon au moins l'une des revendications précédentes, **caractérisé en ce que** l'au moins un système de réception et de liaison (6) est réalisé comme une bande autoagrippante s'étendant dans la direction horizontale.

8. Dispositif de mesure de poids (1) selon au moins l'une des revendications précédentes, **caractérisé en ce que** l'au moins un système de réception et de liaison (6) est formé comme un élément magnétique ou ferromagnétique s'étendant dans la direction horizontale.

9. Dispositif de mesure de poids (1) selon au moins l'une des revendications précédentes, **caractérisé en ce que** ledit dispositif comporte un système de mesure de longueur (7).

10. Dispositif de mesure de poids (1) selon la revendication 9, **caractérisé en ce que** le système de mesure de longueur (7) est formé comme une barre de mesure de longueur.

11. Dispositif de mesure de poids (1) selon au moins l'une des revendications 9 et 10, **caractérisé en ce que** le système de mesure de longueur (7) peut être positionné verticalement.

12. Dispositif de mesure de poids (1) selon au moins l'une des revendications 9 à 11, **caractérisé en ce que** le système de mesure de longueur (7) comporte une butée de tête (7a).

13. Dispositif de mesure de poids (1) selon au moins l'une des revendications précédentes, **caractérisé en ce que** ledit dispositif est formé comme un pèse-personne à colonne.
